(19) Europäisches Patentamt
European Patent Office
Office européen des brevets

(11) Publication number: **0 043 594**
**B1**

(12) **EUROPEAN PATENT SPECIFICATION**

(45) Date of publication of patent specification: **11.05.83**

(51) Int. Cl.³: **C 07 F 7/18**

(21) Application number: **81105627.4**

(22) Date of filing: **08.12.78**

(60) Publication number of the earlier application in accordance with Art. 76 EPC: **0002590**

# (54) 1,1,2-Tris-trimethylsilyloxyethylene and process for its preparation.

(30) Priority: **08.12.77 US 858504**

(43) Date of publication of application: **13.01.82 Bulletin 82/2**

(45) Publication of the grant of the patent: **11.05.83 Bulletin 83/19**

(84) Designated Contracting States: **BE CH DE FR GB IT NL SE**

(56) References cited:
**COLLECTION OF CZECHOSLOVAK CHEMICAL COMMUNICATIONS, vol. 42, 1977, J. SCHRAML et al.: "Application of 29Si-NMR to analysis of silylated amino and other carbonfunctional carboxylic acids", pages 1165—9**

**TETRAHEDRON LETTERS, no. 31, July 1978, A. WISSNER: "Tristrimethylsilyloxyethylene: the conversion of carboxylic acid chlorides to hydroxymethylketones" pages 2749—52**

(73) Proprietor: **AMERICAN CYANAMID COMPANY
Berdan Avenue
Wayne New Jersey 06904 (US)**

(72) Inventor: **Wissner, Allan
31 Wood Avenue
Ardsley New York (US)**

(74) Representative: **Allam, Peter Clerk et al,
LLOYD WISE, TREGEAR & CO. Norman House
105-109 Strand
London WC2R 0AE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 043 594 B1

# 1,1,2-*tris*-trimethylsilyloxyethylene and process for its preparation

This invention relates to a novel compound and to a process for its preparation.

The novel compound of this invention is 1,1,2-*tris*-trimethylsilyloxyethylene, of the formula:

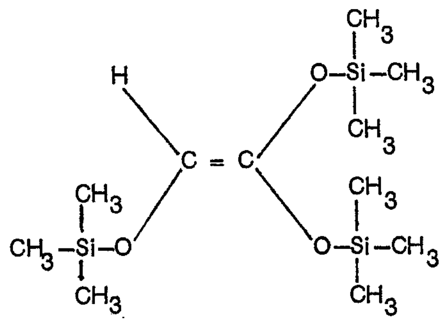

The compound of this invention may be prepared as shown in the Flowsheet A below:

FLOWSHEET A

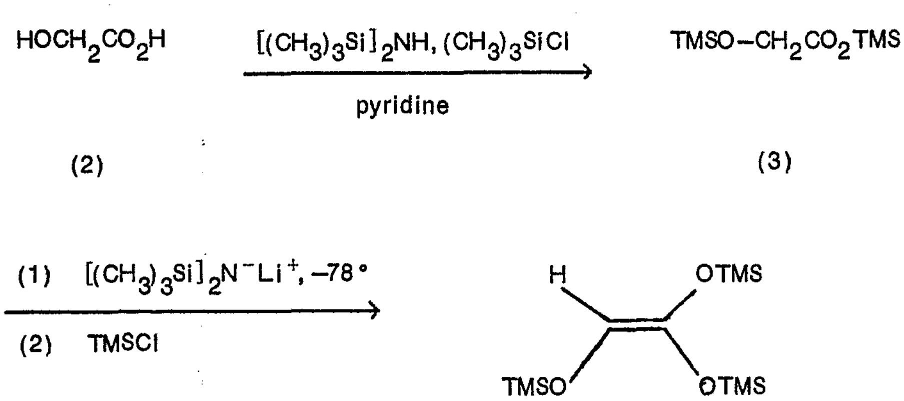


(2) (3)

(1)

As shown in Flowsheet A, the reaction of glycolic acid (2) with 1,1,1,3,3,3-hexamethyldisilazane of the formula $[(CH_3)_3Si]_2NH$ and trimethylsilylchloride in pyridine gives *bis*-trimethylsilated glycolic acid or trimethylsilyl 2-trimethylsilyloxyacetate (3). Addition of (3) to a tetrahydrofuran solution of one equivalent of lithium 1,1,1,3,3,3-hexamethyldisilazane amide at –78°C generates a lithium enolate which is trapped with trimethylsilylchloride to produce the desired compound of this invention (1).

The compound of this invention is useful for converting acid chlorides to hydroxyketones, as is described in our co-pending European Patent Application No. 78300772.7 (Publication No. 0002590A) from which the present application is divided, and as is exemplified in Flowsheets B and C below:

FLOWSHEET B

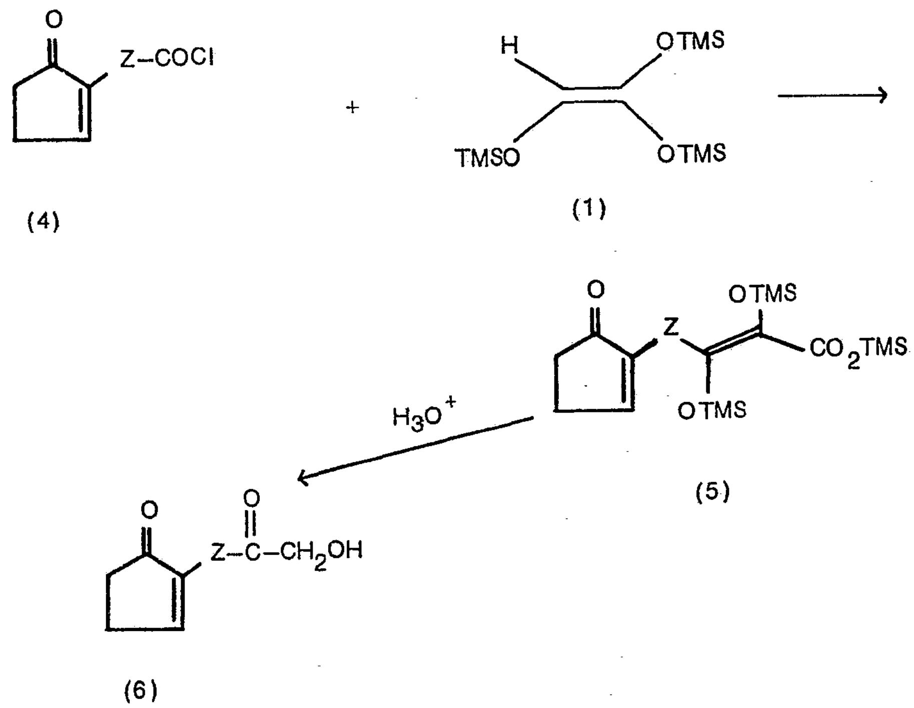


(4) (1) (5) (6)

In accordance with Flowsheet B, cyclopentenone acid chloride (4), wherein Z is $-(CH_2)_g-$ or $-CH_2-CH{=}CH-(CH_2)_f-$ wherein *g* is an integer from 5 to 7 inclusive and *f* is an integer from 2 to 4 inclusive, is heated with two equivalents of 1,1,2-*tris*-trimethylsilyloxyethylene (1) of this invention at 90°—100°C for 2 to 4 hours to produce compound (5). Compound (5) is then readily hydrolyzed and decarboxylated to give the hydroxyketone (6) by treatment with dilute hydrochloric acid in tetrahydrofuran.

FLOWSHEET C

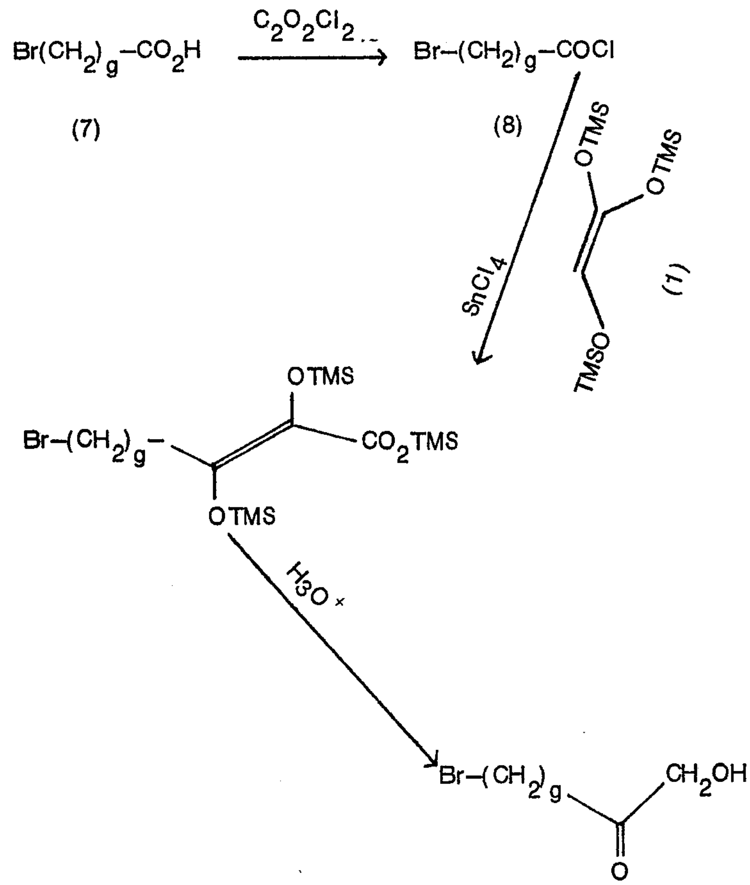


(10)

wherein *g* is as defined above. According to Flowsheet C, $\omega$-bromo-carboxylic acid (7) is reacted with oxalyl chloride in an inert solvent such as benzene to give the acid chloride (8). Acid chloride (8) is then treated with an excess of 1,1,2-*tris*-trimethylsilyloxyethylene (1) of this invention in the presence of a catalytic amount of stannic chloride in the absence of solvent to give compound (9), which can then be readily hydrolyzed and decarboxylated to the desired hydroxyketone (10) using, again, dilute hydrochloric acid in tetrahydrofuran.

The invention is illustrated by the Examples which follow, in which Examples 1 and 2 relate to the preparation of the compound of this invention, and Example 3 relates to the use of this compound to convert an acid chloride to a hydroxy ketone.

Example 1

Preparation of trimethylsilyl 2-trimethylsilyloxyacetate

To a solution of 15 g (0.197 mol) of glycolic acid in 50 ml of dry pyridine is poured 32.3 g (0.2 mol) of 1,1,1,3,3,3-hexamethyldisilazane. After stirring 15 minutes, 10.86 g (0.1 mol) of trimethylsilyl chloride is added dropwise. The mixture is stirred for one hour and then filtered from a white solid which is washed with petroleum ether. The filtrate and washings are concentrated at reduced pressure at 30°C. The residue is distilled (85°—86°C, 15 minutes) to give 38 g of the title compound.

Example 2

Preparation of *tris*-trimethylsilyloxyethylene

To a solution of 50.98 g (0.316 mol) of 1,1,1,3,3,3-hexamethyldisilazane in 250 ml of tetrahydrofuran is added with stirring under argon at 0°C dropwise 133.3 ml (0.32 mol) of 2.4M *n*-butyl lithium in hexane. After addition is complete, the solution is maintained at 45°C for 30 minutes. The solution is cooled to —78°, and 58.7 g of trimethylsilyl 2-trimethylsilyloxyacetate (Example 1) is added dropwise. After stirring 30 minutes, 43.2 g (0.4 mol) of trimethylsilylchloride is added over 10 minutes. The solution is allowed to warm to room temperature over 30 minutes. The solvent is removed at

reduced pressure. The residue is mixed with an equal volume of petroleum ether and filtered from the suspended lithium chloride. The solvent is removed and the residue is distilled (70°—75°C, 1.4 minute) to give 64.65 g of the title compound.

Example 3

Preparation of 2-(8-hydroxy-7-oxo-octyl)cyclopent-2-en-1-one

A mixture of 6.3 g of 2-(6-(chloroformyl)hexyl)cyclopent-2-en-1-one and 16 g of *tris*-trimethylsilyloxyethylene (Example 2) are stirred at 90° to 100°C under argon for one hour. To this mixture is added 25 ml of dioxane and 10 ml of 0.6N hydrochloric acid. The mixture is heated at 80°C for 30 minutes. The mixture is poured into brine and extracted with ether. The ether solution is washed with saturated sodium bicarbonate and dried over magnesium sulfate. The solvent is removed and the residue is chromatographed on a dry column of silica gel eluting with ether containing 2% acetic acid to give 1.7 g of the title compound ($R_f$ = 0.45).

## Claims

1. 1,1,2-*Tris*-trimethylsilyloxyethylene, of the formula:

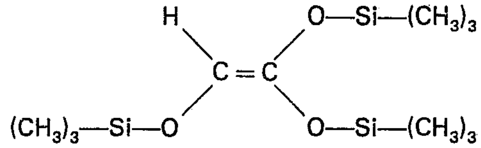

2. A process for the preparation of 1,1,2-*tris*-trimethylsilyloxyethylene which comprises the steps of:

(i) reacting glycolic acid with 1,1,1,3,3,3-hexamethyldisilazane and trimethylsilylchloride in pyridine, to give *bis*-trimethylsilated glycolic acid; and

(ii) adding the product of step (i) to a tetrahydrofuran solution of one equivalent of lithium 1,1,1,3,3,3-hexamethyldisilazane amide at −78°C, followed by addition of trimethylsilylchloride.

## Patentansprüche

1. 1,1,2-*Tris*-trimethylsilyloxyäthylen der Formel

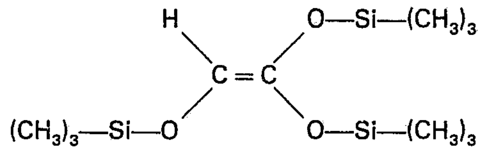

2. Verfahren zur Herstellung von 1,1,2-*Tris*-trimethylsilyloxyäthylen, gekennzeichnet durch die Verfahrensstufen:

(i) der Umsetzung von Glykolsäure mit 1,1,1,3,3,3-Hexamethyldisilazan und Trimethylsilylchlorid in Pyridin, um *bis*-trimethylsilylierte Glykolsäure zu erhalten; und

(ii) die Zugabe des Produkts der Verfahrensstufe (i) zu einer Tetrahydrofuranlösung von einem Äquivalent Lithium-1,1,1,3,3,3-hexamethyldisilazanamid bei −78°C, gefolgt von der Zugabe von Trimethylsilylchlorid.

## Revendications

1. 1,1,2-tris-triméthylsilyloxyéthylène de formule:

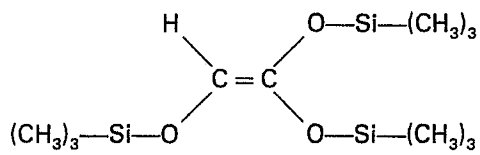

2. Procédé pour la préparation de 1,1,2-tris-triméthylsilyloxyéthylène qui comprend les étapes suivantes:

(i) réaction de l'acide glycolique avec le 1,1,1,3,3,3-hexaméthyldisilazane et le chlorure de triméthylsilyle dans la pyridine pour donner l'acide glycolique bis-triméthylsilé et

(ii) addition de produit de l'étape (i) à une solution dans le tétrahydrofuranne d'un équivalent de 1,1,1,3,3,3-hexaméthyldisilazane amidure de lithium à −78°C, puis addition de chlorure de triméthylsilyle.